# EUROPEAN PATENT APPLICATION

(11) **EP 4 613 218 A1**
(43) Date of publication of application: **10.09.2025**
(21) Application number: 23884688.5
(22) Date of filing: 25.10.2023
(51) Int. Cl.: A61B 17/326

(54) **DISPOSABLE CIRCUMCISION ANASTOMAT**

(30) Priority: 31.10.2022 CN 202211351572; 31.10.2022 CN 202211351557; 28.12.2022 CN 202211698441
(71) Applicant: Wuhu Snnda Medical Treatment Appliance Technology Co., Ltd., Wuhu, Anhui 241001 (CN)
(72) Inventor: SHANG, Jianzhong, Wuhu, Anhui 241001 (CN); SHANG, Jingjing, Wuhu, Anhui 241001 (CN)
(74) Representative: Stanners, David Ralph
(86) International application number: PCT/CN2023/126493
(87) International publication number: WO 2024/093756

(57) **Abstract**

Disclosed is a disposable circumcision anastomat, including an outer ring (1) and an inner ring (2) that match each other. The outer ring (1) is provided with a blunt blade (11, 106) for squeezing a prepuce. An edge (12, 109) of the blunt blade (11, 106) is provided with a plurality of protrusions (13, 110). The inner ring (2) is provided with first pits (21, 201) at positions corresponding to the protrusions (13, 110). The outer ring (1) is of a structure of which two ends are openable and closed in a snap-fit manner. A first binding block (17A, 101) and a second binding block (17B, 102) that match each other are provided at the two ends of the outer ring (1) respectively. At least one of the first binding block (17A, 101) and the second binding block (17B, 102) is provided with an elastic groove (18, 104). A plurality of pits (21, 201) are circumferentially arranged on an inner side of the blunt blade (11, 106). The disposable circumcision anastomat further achieves the effect of avoiding edema and accelerates healing of a preputial wound; it is easier to close and disassemble and more convenient to use the outer ring; and the function of lymph drainage is achieved by structural improvement.

## Description

### TECHNICAL FIELD

The present disclosure relates to the technical field of circumcision instruments and in particular to a disposable circumcision anastomat.

### BACKGROUND ART

Redundant prepuce or phimosis is one of the root causes of male urinary tract infections and sexually transmitted diseases. Redundant prepuce or phimosis may cause urinary tract infections and hence result in chronic prostatitis and a series of symptoms such as backache, impotence and premature ejaculation. Therefore, the removal of redundant prepuce or phimosis is one of the good measures to prevent these diseases. In addition, the implementation of circumcision can effectively reduce the risk of AIDS transmission, the role of circumcision in the process of AIDS prevention has gradually become prominent, and thus circumcision has won admiration in many countries. The series of circumcision anastomat products invented by the inventors of the present disclosure have been listed as key products and technologies promoted by authoritative departments, and the development of circumcision anastomats has entered a new stage of development and promotion.

An existing disposable circumcision anastomat is, for example, the Chinese invention patent with grant announcement number of CN102327141B previously filed by the inventors.

In an embodiment, an inner ring and an outer ring are included. The outer ring is provided with an opening end, and the opening end is connected by a fixing device. A blade structure located at two side of the opening end is respectively provided with an upper half blade extension joint and a lower half blade extension joint. The upper half blade extension joint and the lower half blade extension joint are formed at two ends of an opening of the outer ring and extend towards outer ends by certain extended segments. Hanging teeth are arranged on each blade on the outer ring, the hanging teeth may be protrusions with arc-shaped outer sides, and the hanging teeth serve to relieve postoperative edema symptoms and prevent prepuce movement from damaging the surgery effect during the operation. Through further research by the inventors, it was found that the hanging teeth in this solution can well fix the prepuce and prevent the prepuce from moving, but the prepuce is in a completely closed state between the blade, the hanging teeth and the inner ring, and thus it is still impossible to avoid the situation that the prepuce is prone to edema. Other conventional technologies have failed to give a solution to avoid edema caused by the circumcision anastomat. Edema may lead to slow healing of a preputial wound, cause great psychological pressure to a patient, and may also result in more serious infections, complications, etc. Therefore, a new circumcision anastomat is needed to solve this problem.

In an embodiment, an inner ring and an outer ring are included. The outer ring is provided with an opening end, and the opening end is connected by a fixing device. The fixing device includes a first serrated snap-fit block and a second serrated snap-fit block arranged at two ends of an opening, and the first serrated snap-fit block and the second serrated snap-fit block are fixed by engaging with each other. Through further research by the inventors, it was found that in this solution, the fixing device has a good fixing effect, but since the outer ring is generally made of integrally molded hard plastic, it is difficult or even impossible to close and disassemble the first serrated snap-fit block and the second serrated snap-fit block, which leads to inconvenience in use. Therefore, a new circumcision anastomat is needed to solve this problem.

The existing disposable circumcision anastomat, for example, the China utility model patent with the grant announcement number of CN201710426U previously filed by the inventors, includes an inner ring and an outer ring. The outer ring is provided with a blade structure, so as to circumcise and occlude a prepuce. Through further research by the inventors, it was found that this solution and other existing circumcision anastomats cannot avoid the situation that the prepuce is prone to edema due to lymph accumulation. Edema may lead to slow healing of a preputial wound, cause great psychological pressure to a patient, and may also result in more serious infections, complications, etc. Therefore, a new circumcision anastomat is needed to solve this problem.

### SUMMARY OF THE INVENTION

The present disclosure solves the following technical problems: providing a disposable circumcision anastomat to further achieve the effect of avoiding edema and accelerate healing of a preputial wound; making it easier to close and disassemble an outer ring and more convenient to use the outer ring by structural improvement; and achieving the function of lymph drainage by structural improvement to achieve the effect of effectively avoiding edema.

In order to fulfill the above objectives, the present disclosure adopts the following technical solution I: a disposable circumcision anastomat, including an outer ring and an inner ring that match each other, the outer ring being provided with a blunt blade for squeezing a prepuce, and an edge of the blunt blade being provided with a plurality of protrusions, wherein the inner ring is provided with first pits at positions corresponding to the protrusions. The first pits and the protrusions on the blunt blade match each other in one-to-one correspondence to penetrate through inner and outer preputial layers, such that lymph between the inner and outer preputial layers may exude, which is not prone to edema and accelerates healing of a preputial wound.

In an embodiment, the first pits are through holes.

In another embodiment, a spring gasket is arranged on an outer side of the inner ring, and the spring gasket is provided with second pits at positions corresponding to the protrusions. The spring gasket is for example made of a rubber material, which has greater friction and better stability.

Further optionally, the second pits are through holes.

Preferably, additional bumps are added above and/or below the edge of the blunt blade.

Further, heights of the additional bumps are greater than a thickness of the edge of the blunt blade.

Preferably, the additional bumps have a same shape as the protrusions and are triangular, conical, polygonal, regular quadrilateral or U-shaped.

In yet another preferred embodiment, a spring gasket is arranged on the outer side of the inner ring, the spring gasket is provided with second pits at positions corresponding to the protrusions, and the additional bumps and the protrusions are inserted into the second pits of the spring gasket or protrude out of the second pits of the spring gasket.

Further, the outer ring is of an integral C-shaped structure made of a soft material, or is of a movably connected two-segment or multi-segment structure.

Preferably, at least one circle of annular blunt blade is arranged on an inner side of the outer ring, an upper half blade extension and a lower half blade extension that match each other are arranged at two ends of an opening of the outer ring, and the upper half blade extension and the lower half blade extension have rounded corners at outward end portions.

Preferably, a first binding block and a second binding block that match each other are provided at two ends of the outer ring respectively, and at least one of the first binding block and the second binding block is provided with an elastic groove.

Preferably, the inner ring is of either an integral annular structure or a two-segment or multi-segment arc-shaped structure.

In some other embodiments, at least two layers of blunt blades are arranged on the inner side of the outer ring.

Further, the disposable circumcision anastomat is circular or oval.

Preferably, an upper side face and a lower side face of the blunt blade of the outer ring are frosted faces.

Preferably, the outer ring is provided with protrusion indicators at positions corresponding to the protrusions, and/or the inner ring is provided with pit indicators at positions corresponding to the first pits.

In order to fulfill the above objectives, the present disclosure adopts the following technical solution II: a disposable circumcision anastomat, including an outer ring and an inner ring that match each other, the outer ring being of a structure of which two ends are openable and closed in a snap-fit manner, wherein a first binding block and a second binding block that match each other are provided at two ends of the outer ring respectively, and at least one of the first binding block and the second binding block is provided with an elastic groove.

Further, the first binding block is located at an upper portion of the outer ring and the second binding block is located at a lower portion of the outer ring; snap-fit portions that match each other are arranged on opposite side faces of the first binding block and the second binding block; and the elastic groove is located on an inward side of the first binding block or the second binding block, and runs through the first binding block or the second binding block lengthwise, and an elastic connecting plate is formed between the snap-fit portions and the two ends of the outer ring.

Preferably, the elastic connecting plate in the first binding block is located above, and the elastic connecting plate in the second binding block is located below.

Preferably, the snap-fit portion is stepped and serrated.

According to some embodiments, the outer ring is of an integral C-shaped structure made of a soft material, or is of a movably connected two-segment or multi-segment structure.

Further, at least one circle of annular blunt blade is arranged on an inner side of the outer ring, an upper half blade extension and a lower half blade extension that match each other are arranged at the two ends of the outer ring, and the upper half blade extension and the lower half blade extension have rounded corners at outward end portions.

Preferably, an inner side of the blunt blade of the outer ring is an edge, the edge is provided with a plurality of protrusions, and the inner ring is provided with first pits at positions corresponding to the protrusions.

Preferably, a spring gasket is arranged on an outer side of the inner ring, and the spring gasket is provided with second pits at positions corresponding to the protrusions.

Optionally, the first pits and/or the second pits are through holes.

Preferably, additional bumps are added above and/or below the edge.

Preferably, the outer ring is provided with protrusion indicators at positions corresponding to the protrusions, and/or the inner ring is provided with pit indicators at positions corresponding to the pits.

According to some embodiments, the inner ring is of either an integral annular structure or a two-segment or multi-segment arc-shaped structure.

According to some embodiments, at least two layers of blunt blades are arranged on the inner side of the outer ring.

According to some embodiments, the disposable circumcision anastomat is circular or oval.

Preferably, an upper side face and a lower side face of the blunt blade of the outer ring are frosted faces.

In order to fulfill the above objectives, the present disclosure adopts the following technical solution III: a disposable circumcision anastomat, including an outer ring and an inner ring that match each other, the outer ring being provided with a blunt blade for squeezing a prepuce, wherein a plurality of pits are circumferentially arranged on an inner side of the blunt blade.

Preferably, the pits are either circular point-like pits or longitudinal strip-shaped grooves.

Preferably, the pits have a depth of 2 mm.

Preferably, a plurality of protrusions are arranged on an outer side of the inner ring, and the protrusions and the pits are in one-to-one correspondence.

Preferably, a spring gasket is arranged on the outer side of the inner ring.

Preferably, the spring gasket is provided with gasket pits at positions corresponding to the pits.

Preferably, the gasket pits are through holes.

According to some embodiments, the outer ring is of an integral C-shaped structure made of a soft material, or is of a movably connected two-segment or multi-segment structure.

According to some embodiments, at least one circle of annular blunt blade is arranged on an inner side of the outer ring, an upper half blade extension and a lower half blade extension that match each other are arranged at two ends of an opening of the outer ring, and the upper half blade extension and the lower half blade extension have rounded corners at outward end portions.

According to some embodiments, a first binding block and a second binding block that match each other are provided at two ends of the outer ring respectively, and at least one of the first binding block and the second binding block is provided with an elastic groove.

According to some embodiments, the inner ring is of either an integral annular structure or a two-segment or multi-segment arc-shaped structure.

According to some embodiments, at least two layers of blunt blades are arranged on the inner side of the outer ring.

According to some embodiments, the disposable circumcision anastomat is circular or oval.

According to some embodiments, an upper side face and a lower side face of the blunt blade of the outer ring are frosted faces.

According to some embodiments, the outer ring and/or the inner ring is/are provided with indicators at positions corresponding to the pits.

The disposable circumcision anastomat adopting the technical solution I of the present disclosure at least has the following beneficial effects.
1. When in use, the disposable circumcision anastomat according to the present disclosure can penetrate through inner and outer preputial layers, such that lymph between the inner and outer preputial layers may exude, which is not prone to edema and accelerates healing of a preputial wound.
2. The disposable circumcision anastomat according to the present disclosure is preferably provided with the additional bumps, and point contact of the protrusions is changed into line or face contact, to better penetrate through the inner and outer preputial layers and to be more convenient for lymph to exude.
3. In the disposable circumcision anastomat according to the present disclosure, the elastic grooves are preferably arranged on the first binding block and the second binding block, such that it is easier to snap-fit and fix the outer ring, and it is also easier and more convenient to install and use the outer ring.
4. In the disposable circumcision anastomat according to the present disclosure, the inner ring is preferably of a two-segment or multi-segment structure, which makes it easier to install and disassemble and more comfortable in use.

The disposable circumcision anastomat adopting the technical solution II of the present disclosure at least has the following beneficial effects.
1. In the disposable circumcision anastomat according to the present disclosure, the elastic grooves are preferably arranged on the first binding block and the second binding block, such that the elastic connecting plate is formed between the snap-fit portion and the two ends of the outer ring. Compared with the solutions in the related art, this solution can make it easier, by means of the elastic connecting plate, for the snap-fit portion to elastically deform on the premise of ensuring the fixing effect of the outer ring, such that it is easier to snap-fit, fix and disassemble the outer ring, and it is also easier and more convenient to install and use the outer ring.
2. The disposable circumcision anastomat according to the present disclosure preferably has the protrusions on the outer ring and the pits on the inner ring. Therefore, when in use, the disposable circumcision anastomat can penetrate through inner and outer preputial layers, such that lymph between the inner and outer preputial layers may exude, which is not prone to edema and accelerates healing of a preputial wound.
3. In the disposable circumcision anastomat according to the present disclosure, the inner ring is preferably of a two-segment or multi-segment structure, which makes it easier to install and disassemble and more comfortable in use.

The disposable circumcision anastomat adopting the technical solution III of the present disclosure at least has the following beneficial effects.
1. In the disposable circumcision anastomat according to the present disclosure, the pits are formed on the inner side of the blunt blade of the outer ring to play a role of draining lymph, such that edema can be effectively prevented and healing of a preputial wound can be accelerated.
2. The disposable circumcision anastomat according to the present disclosure preferably has the protrusions on the inner ring. Therefore, when in use, the disposable circumcision anastomat can penetrate through inner and outer preputial layers, such that lymph between the inner and outer preputial layers may exude, which further prevents lymph from accumulation.
3. In the disposable circumcision anastomat according to the present disclosure, the inner ring is preferably of a two-segment or multi-segment structure, which makes it easier to install and disassemble and more comfortable in use.

### BRIEF DESCRIPTION OF THE DRAWINGS

In solution I:
FIG. 1 is a schematic diagram of an overall three-dimensional structure according to an embodiment of the present disclosure;
FIGS. 2A and 2B are schematic diagrams of sectional structures according to some embodiments of the present disclosure;
FIGS. 3A to 3D are schematic sectional views of a binding portion between an inner ring and an outer ring according to some other embodiments of the present disclosure;
FIG. 4 is a schematic sectional view of the binding portion between the inner ring and the outer ring according to another embodiment of the present disclosure;
FIGS. 5A and 5B are schematic diagrams of a top-view structure of the outer ring according to some embodiments of the present disclosure;
FIG. 6 is a schematic diagram of a three-dimensional structure of the outer ring according to yet another embodiment of the present disclosure;
FIG. 7 is a schematic sectional view of the binding portion between the inner ring and the outer ring according to still yet another embodiment of the present disclosure;
FIG. 8A is a schematic diagram of a three-dimensional structure of the inner ring according to still yet another embodiment of the present disclosure;
FIG. 8B is a schematic sectional view of the inner ring shown in FIG. 8A; and
FIG. 9 is a schematic diagram of a top-view structure of the outer ring and the inner ring according to still yet another embodiment of the present disclosure.

In solution II:

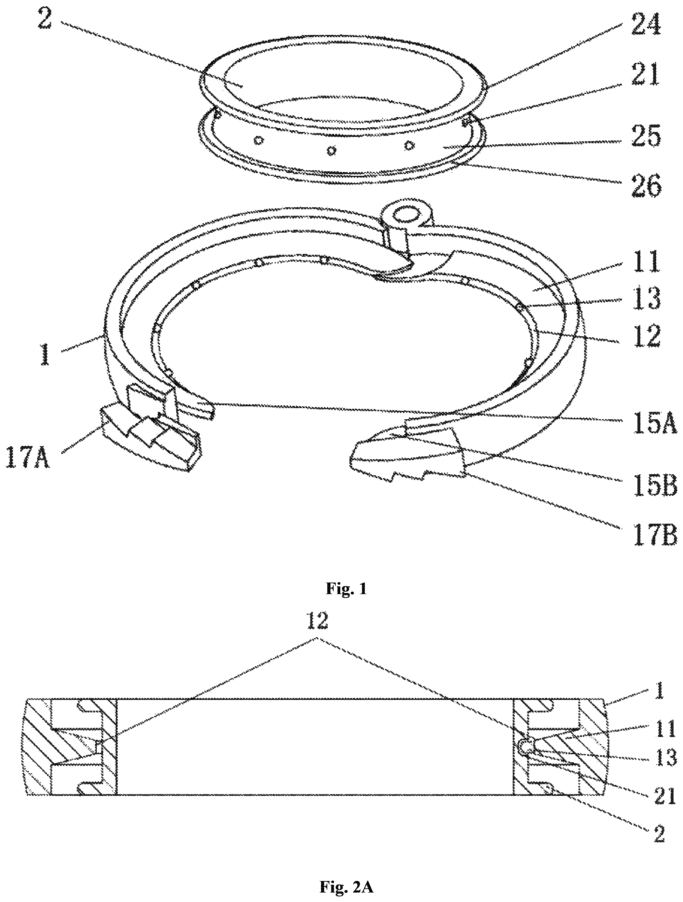

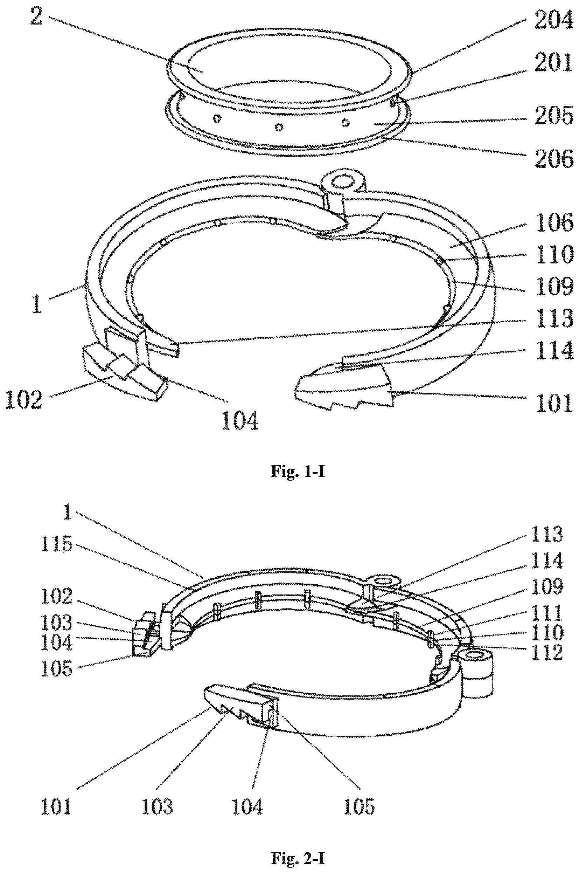

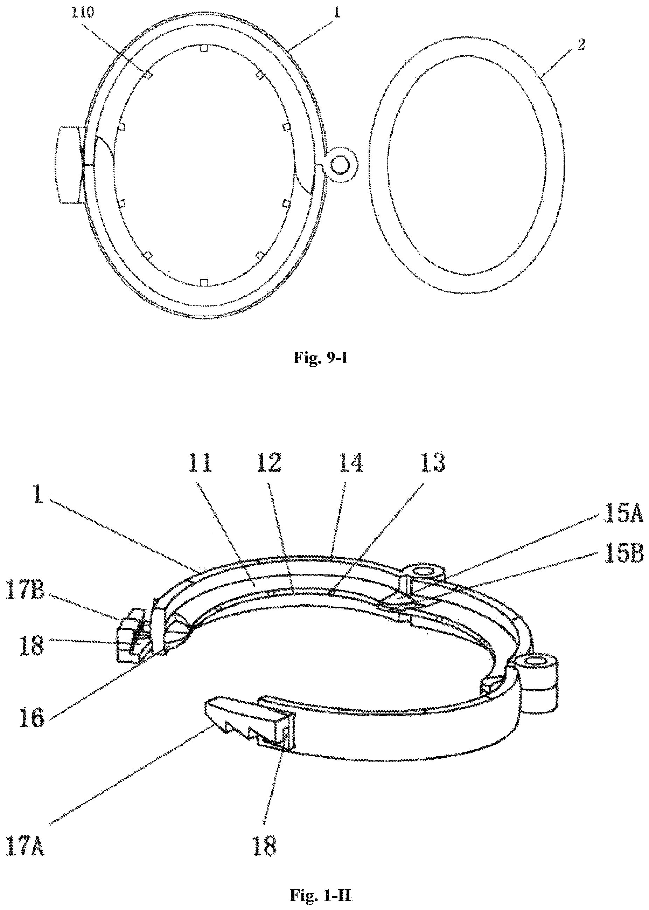

FIG. 1-I is a schematic diagram of an overall three-dimensional structure according to an embodiment of the present disclosure;

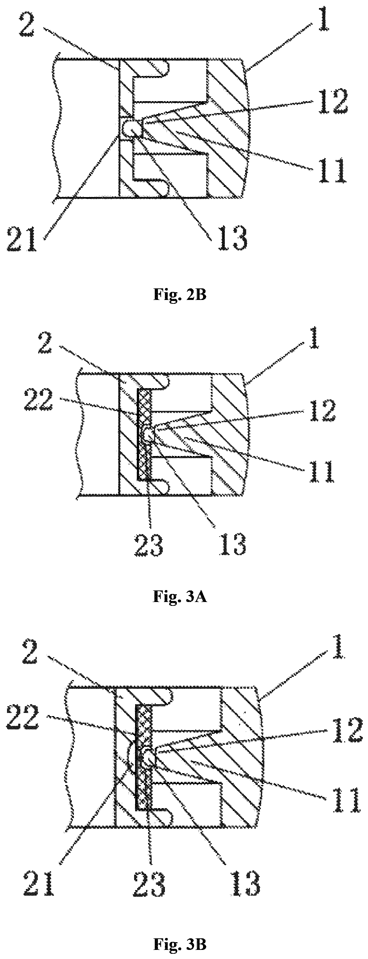

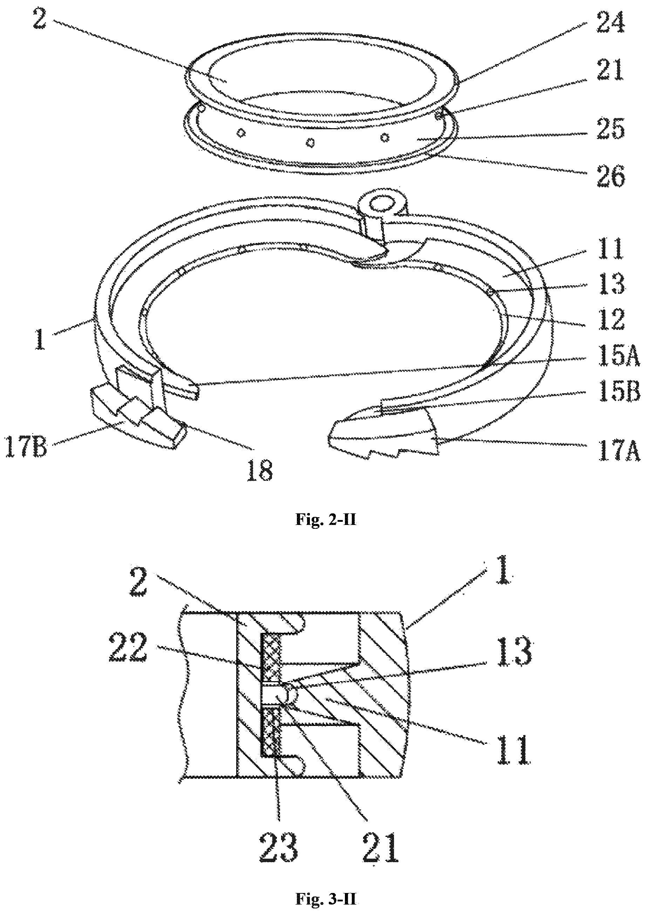

FIG. 2-I is a schematic structural diagram of an outer ring according to another embodiment of the present disclosure;

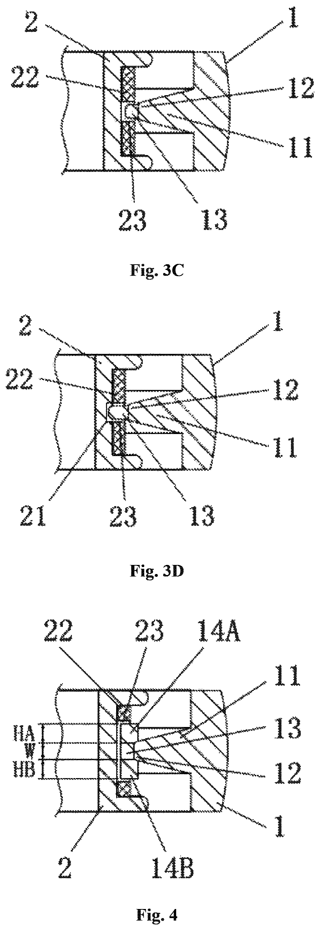

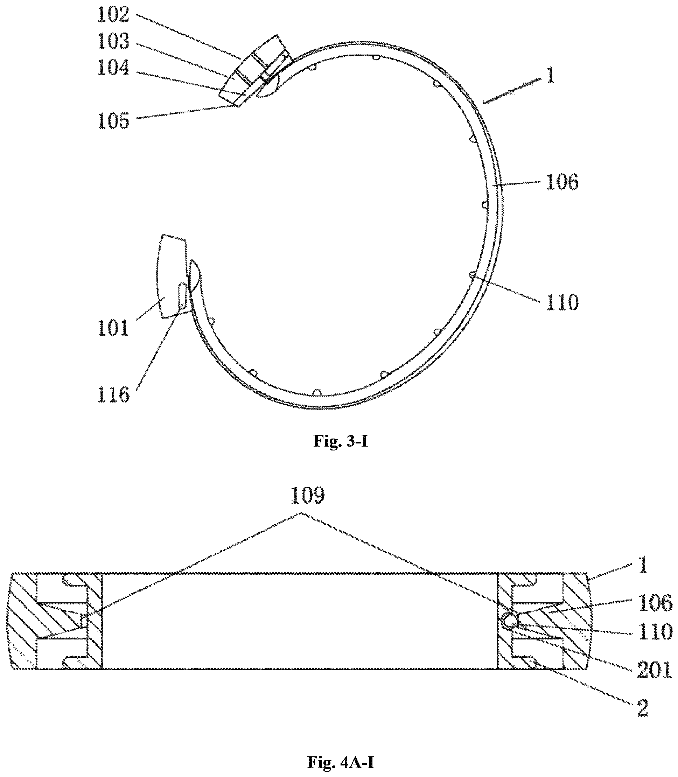

FIG. 3-I is a schematic structural diagram of the outer ring according to yet another embodiment of the present disclosure;

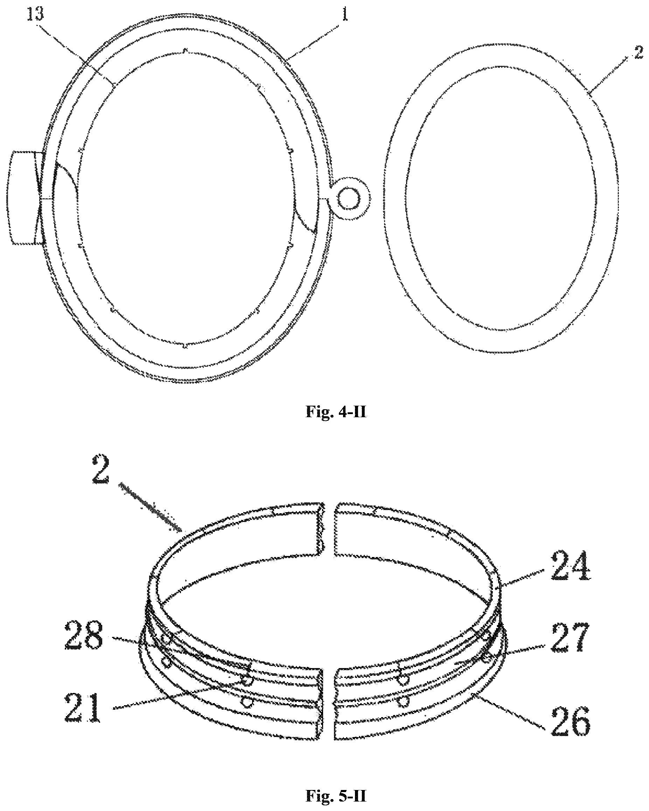

FIGS. 4A-I and 4B-I are schematic sectional views of a binding portion between an inner ring and the outer ring according to some embodiments of the present disclosure;

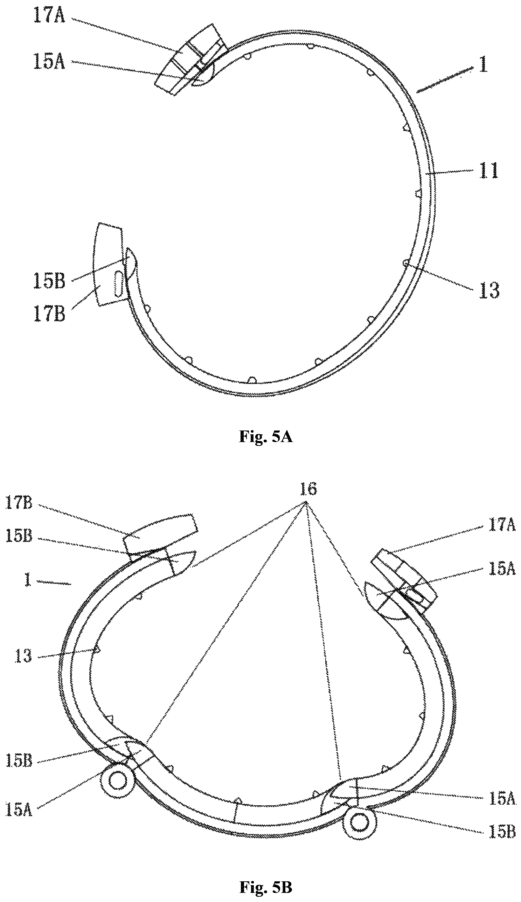

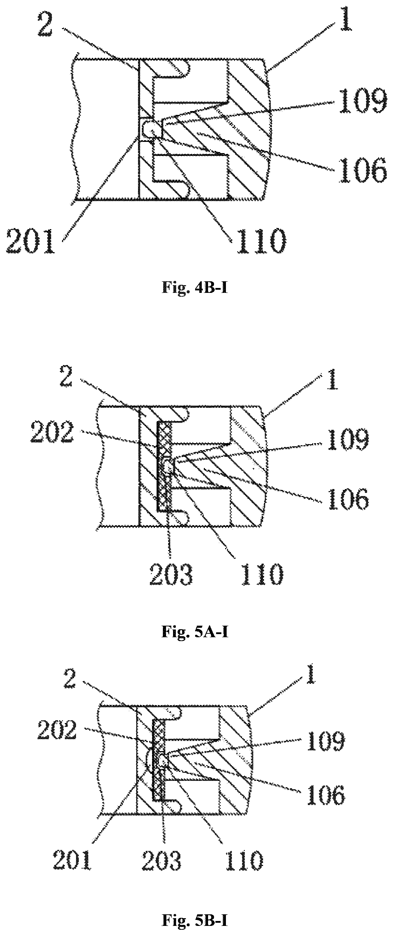

FIGS. 5A-I to 5D-I are schematic sectional views of the binding portion between the inner ring and the outer ring according to some other embodiments of the present disclosure;

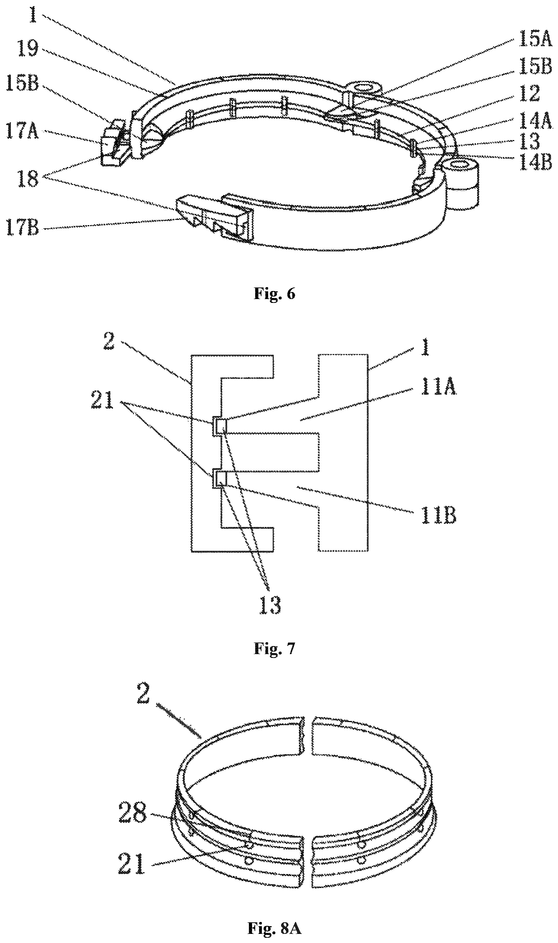

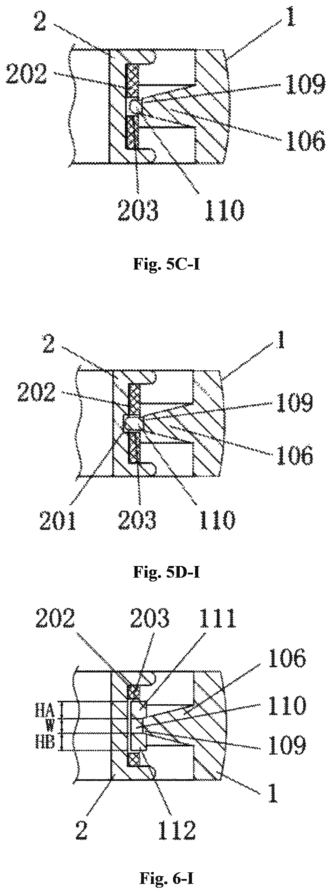

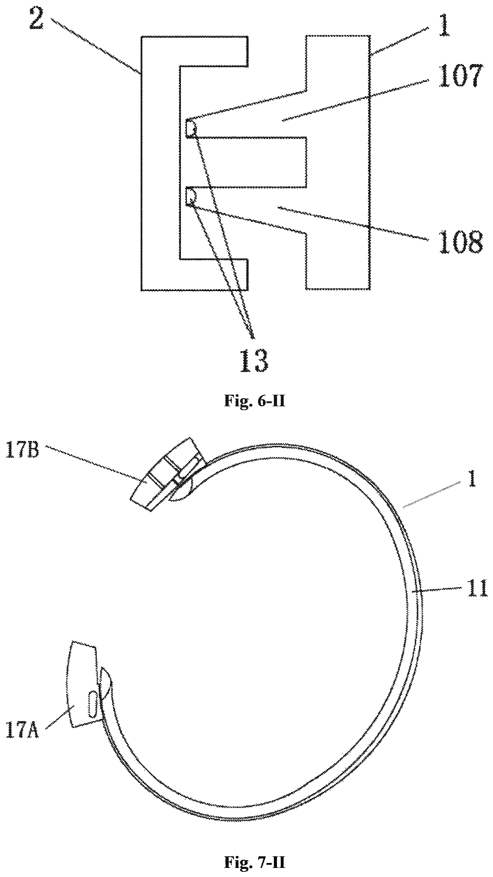

FIG. 6-I is a schematic sectional view of the binding portion between the inner ring and the outer ring according to still yet another embodiment of the present disclosure;

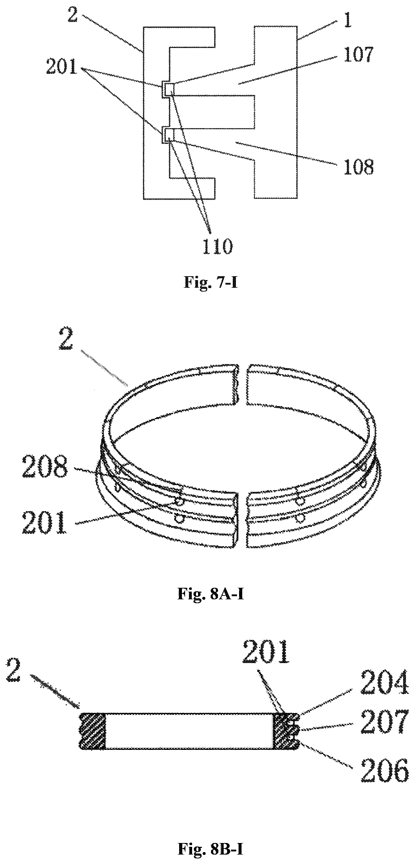

FIG. 7-I is a schematic sectional view of the binding portion between the inner ring and the outer ring according to still yet another embodiment of the present disclosure;
FIG. 8A-I is a schematic diagram of a three-dimensional structure of the inner ring according to still yet another embodiment of the present disclosure;

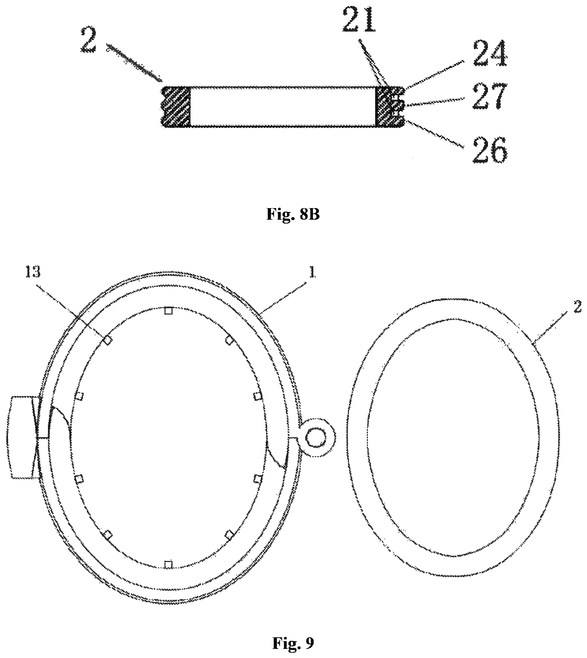

FIG. 8B-I is a schematic sectional view of the inner ring shown in FIG. 8A-I; and
FIG. 9-I is a schematic diagram of a top-view structure of the outer ring and the inner ring according to still yet another embodiment of the present disclosure.

In solution III:
FIG. 1-II is a schematic diagram of a three-dimensional structure of an outer ring according to an embodiment of the present disclosure;
FIG. 2-II is a schematic diagram of a three-dimensional structure of an inner ring and the outer ring according to another embodiment of the present disclosure;
FIG. 3-II is a schematic sectional view of a binding portion between the inner ring and the outer ring according to yet another embodiment of the present disclosure;
FIG. 4-II is a schematic diagram of a top-view structure of the outer ring and the inner ring according to still yet another embodiment of the present disclosure;
FIG. 5-II is a schematic diagram of a three-dimensional structure of the inner ring according to still yet another embodiment of the present disclosure;
FIG. 6-II is a schematic sectional view of the binding portion between the inner ring and the outer ring according to still yet another embodiment of the present disclosure; and
FIG. 7-II is a schematic diagram of a top-view structure of the outer ring according to still yet another embodiment of the present disclosure.

### DETAILED DESCRIPTION

In solution I of the present disclosure, a disposal circumcise anastomat is provided, which, by improving the structural designs of an inner ring and of protrusions on an inner side of a blunt blade, achieves the effect of effectively avoiding edema and can accelerate healing of a preputial wound.

Referring to FIG. 1, a disposable circumcision anastomat according to an embodiment of the present disclosure includes an outer ring 1 and an inner ring 2 that match each other, and a blunt blade 11 for squeezing a prepuce is arranged on an inner side of the outer ring 1. The blunt blade 11 is of a sheet shape, and the blunt blade 11 forms a ring shape after the outer ring 1 is closed. The thickness of the blunt blade 11 gradually decreases from the outside in, thereby forming an edge 12 on an inner side. The edge 12 is provided with a plurality of inwardly protruding protrusions 13 (e.g., ten protrusions are uniformly distributed, which may be specifically adjusted and designed as required). One of the main improvements of the present disclosure lies in that the inner ring 2 is provided with first pits 21 at positions corresponding to the protrusions 13.

Referring to FIGS. 2A and 2B, the first pits 21 may be blind holes or through holes, and serve to accommodate at least some of the protrusions 13, such that the protrusions 13 can penetrate through inner and outer preputial layers after the outer ring 1 is installed. At a position where there is no protrusion 13 on the blunt blade 11, a certain distance is kept between the edge 12 and the inner ring 2 to form a gap for accommodating the prepuce. In the solutions of the related art, the inner ring has no pits, and the prepuce can only be pushed against a side wall of the inner ring, while the prepuce in the structures of the outer ring and the inner ring is completely closed, which is prone to cause edema. In this solution, the first pits 21 arranged on an outer side of the inner ring 2 and the protrusions 13 on the blunt blade 13 match each other in one-to-one correspondence to penetrate through the inner and outer preputial layers, such that lymph between the inner and outer preputial layers can exude, which is not prone to edema and accelerates healing of a preputial wound.

Further specifically, as shown in FIG. 1, for example, the inner ring 2 is for example of a ring shape and is provided with an upper flange 24 and a lower flange 26 respectively protruding outwards at two ends, and the prepuce is clamped and fixed between an inner ring sidewall 25 therebetween and the blunt blade 11, which is more stable. In some embodiments, the inner ring 2 further includes a spring gasket 22 (the spring gasket 22 and the inner ring 2 may be of a split structure or an integral structure) arranged on the outer side of the inner ring. The spring gasket is for example made of a rubber material, which has greater friction and better stability. The spring gasket is provided with second pits 23 at positions corresponding to the protrusions 13. The second pits 23 may be blind holes or through holes.

It should be noted that the action principle of the second pits 23 is basically the same as that of the first pits 21, and therefore, in the embodiment having the spring gasket 22, the second pits 23 and the first pits 21 may be regarded as the same structure. For example, in the embodiment shown in FIG. 3A, the first pits 21 on the inner ring 2 are the second pits 23 on the spring gasket 22, which is equivalent to setting, based on the embodiment shown in FIG. 2A, an outer side face of the inner ring 2 to be elastic, and the spring gasket 22 and the inner ring 2 to be of an integral structure. Alternatively, as shown in FIG. 3B, based on the embodiment shown in FIG. 3A, the second pits 23 and the first pits 21 are of different structures, and when in use, the spring gasket 22 is pressed, by virtue of its elasticity, into the first pits 21 formed in the body of the inner ring 2. Further, since the spring gasket 22 is relatively thin, it can be envisaged that the second pits 23 are not formed on the spring gasket, and the spring gasket is pressed into the first pits 21 only by virtue of its elasticity, thus omitting the corresponding processing procedure, improving the production efficiency and reducing the cost. Similarly, in the embodiment shown in FIG. 3C, the second pits 23 are through holes, but there is no pit on the body of the inner ring 2, and the second pits 23 form the blind-hole type first pits 21. As shown in FIG. 3D, based on the embodiment shown in FIG. 3C, the body of the inner ring 2 is also separately provided with the first pits 21. As another example, the first pits 21 on the body of the inner ring 2 are further in a form of through holes, etc. These conceivable variants are all available.

Further, referring to FIGS. 6 and 8A, the outer ring 1 is provided with protrusion indicators 19 at positions corresponding to the protrusions 13, and/or the inner ring 2 is provided with pit indicators 28 at positions corresponding to the first pits 21 (or second pits 23). Preferably, both the outer ring 1 and the inner ring 2 have corresponding structures thereon. For the case of the outer ring 1, the protrusion indicators 19 may be arranged on an upper face or outer side face of an outer cylinder and/or an upper face of the blunt blade 11, etc. For the case of the inner ring 2, the pit indicators 28 may be arranged on an upper face and/or outer side face of the upper flange 24, etc. The protrusion indicators 19 and the pit indicators 28 may be in any form of marking, such as a relief form of projections and grooves, or simply a form of carving on the face. The protrusion indicators 19 and the pit indicators 28 play a role of facilitating observation during installation, thereby helping a user to align the positions of the protrusions and pits more quickly.

In a further preferred embodiment of the present disclosure, additional bumps are added above and/or below the edge 12. For example, as shown in FIG. 4, an upper additional bump 14A and a lower additional bump 14B are arranged above the edge 12 and below the protrusions 13, respectively. The shapes of the first pits 21 and/or second pits 23 correspond to the protrusions 13 and the additional bumps. The additional bumps and the protrusions 13 have such a length in a radial direction that they can be inserted into the second pits 23 of the spring gasket 22, or that they can protrude out of the second pits 23 of the spring gasket 22 from the outside in (with their ends entering the first pits 21 in the body of the inner ring 2). That is, compared with the foregoing embodiments, the inward protruding portion of the edge 12 is changed from a point shape to a vertical line or face shape, which better serves to penetrate through the inner and outer preputial layers to promote lymph exuding, thereby solving the problem of edema occurring during the total confinement operation of circumcision. Preferably, the heights of the additional bumps (the height HA of the upper additional bump and the height HB of the lower additional bump) are greater than the thickness W of the edge of the blunt blade and the heights of the protrusions 13, such that the effects of the additional bumps are more significant. Preferably, the additional bumps have the same sectional shape as the protrusions 13 and are triangular (as shown in FIG. 5B), conical, polygonal, regular quadrilateral (as shown in FIGS. 6 and 9) or U-shaped (as shown in FIG. 5A). It should be noted that the additional bumps may include the upper additional bump 14A and the lower additional bump 14B, or either of the two, and the upper additional bump 14A and/or the lower additional bump 14B may be staggered instead of being vertically aligned with the protrusions 13 as shown in FIG. 6.

Further, the outer ring 1 may be of an integral C-shaped structure made of a soft material (such as soft plastic) (as shown in FIG. 5A), and is opened and closed by virtue of its softness; or the outer ring 1 may be of a movably connected two-segment or multi-segment structure (as shown in FIG. 1 and FIG. 5B), with each arc-shaped segment rotatably connected via a pivot structure to achieve opening and closing.

At least one circle of blunt blade 11 is arranged on the inner side of the outer ring 1, and an upper side face and a lower side face of the blunt blade 11 are preferably frosted faces, in order to prevent adhesion. The blunt blade is provided with an upper half blade extension 15A and a lower half blade extension 15B that match each other at two ends of an opening of the outer ring 11 (as well as at a movably connected portion(s) in the case of a two-segment or multi-segment structure). For example, by halving the thickness at a corresponding position, an upper half of the upper half blade extension 15A is only retained, and a lower half of the lower half blade extension 15B is only retained, such that the two can overlap together during the closing of the outer ring 1. Referring to FIG. 5B, the upper half blade extension 15A and the lower half blade extension 15B have rounded corners 16 at outer ends, such that their inner side edges are smoothly curved during opening and closing, and the prepuce will not be accidentally trapped during closing.

A first binding block 17A and a second binding block 17B are arranged at two ends of the outer ring 1, respectively. For example, as shown in FIG. 6, the first and second binding blocks are arranged with a height difference and provided with serrations on their opposing faces. The serrations have a gentler slope on a side opposite to the opening of the outer ring 1 and a steeper slope on a side facing outward, such that they can be snap-fitted and fastened when the opening is closed and will not separate easily (the first binding block 17A and the second binding block 17B can be pried apart by a hard rod-type tool after the use of the disposable circumcision anastomat). Preferably, at least one of the first binding block 17A and the second binding block 17B is provided with an elastic groove 18, which is used to reduce the thickness of the portion(s) of the first binding block 17A and/or second binding block 17B that is/are connected to the outer ring 1, such that the portion(s) is/are more susceptible to elastic deformation, allowing for easier snap-fit when the outer ring 1 is installed and closed. In addition, in order to achieve a better snap-fitting and fixing effect, preferably, the first and second binding blocks and the upper and lower half blade extensions are staggered. That is, as shown in FIG. 1, for example, the first binding block 17A is located below, and the upper half blade extension 15A is then adjacent thereto; and at the other end for binding, the second binding block 17B is located above, and the lower half blade extension 15B is located below. In this way, after the outer ring 1 is closed, the upper half blade extension 15A and the lower half blade extension 15B can simultaneously act as a stopper for the first binding block 17A and the second binding block 17B, thereby preventing slippage and separation. It should be noted that snap-fit structures, bolt-screw hole structures or other structures having similar fixing effect are conceivable, which is not limited in the present disclosure.

In some further embodiments of the present disclosure, referring to FIG. 7, at least two circles of blunt blades (including, for example, an upper blade 11A and a lower blade 11B) are arranged on the inner side of the outer ring 1 to form a double-blade (or multi-blade) structure to strengthen the blocking effect on the prepuce. Moreover, a space having a certain volume is naturally formed between the two or more blades, and the space can be used for holding cotton wool, ointment, or medicinal solution, so as to eliminate inflammation and accelerate healing. For the case where both the upper blade 11A and the lower blade 11B (or more blades) are provided with the protrusions 13, the inner ring 2 is correspondingly provided with two (or more) rows of first pits 21 above and below. Further preferably, as shown in FIGS. 8A and 8B, the inner ring 2 is provided with central projections 27 at positions corresponding to the positions between the two (or more) rows of blades, which form, with the upper flange 24 and the lower flange 26, grooves corresponding to the double blades (or more blades). This further enhances the effect of clamping, fixing and blocking the prepuce to be removed, resulting in necrosis due to poor blood flow.

In another aspect, in addition to the integral annular structure shown in FIG. 1, the inner ring 2 may adopt a two-segment (or multi-segment) arc-shaped structure as shown in FIG. 8A. This avoids the discomfort of a patient caused by the fixed inner diameter of the inner ring 2, and is more convenient for installation and removal.

Furthermore, as shown in FIG. 9, the disposable circumcision anastomat according to the present disclosure may be circular or oval, with the outer ring 1 and the inner ring 2 matching in shape.

In summary, the disposable circumcision anastomat according to the present disclosure at least has the following beneficial effects.
1. When in use, the disposable circumcision anastomat according to the present disclosure can penetrate through inner and outer preputial layers, such that lymph between the inner and outer preputial layers can exude, which is not prone to edema and accelerates healing of a preputial wound.
2. The disposable circumcision anastomat according to the present disclosure is preferably provided with the upper additional bump 14A and the lower additional bump 14B, and point contact of the protrusions 13 is changed into line or face contact, to better penetrate through the inner and outer preputial layers and to be more convenient for lymph to exude.
3. In the disposable circumcision anastomat according to the present disclosure, the elastic grooves are preferably arranged on the first binding block 17A and the second binding block 17B, such that it is easier to snap-fit and fix the outer ring 1, and it is also easier and more convenient to install and use the outer ring.
4. In the disposable circumcision anastomat according to the present disclosure, the inner ring 2 is preferably of a two-segment or multi-segment structure, which makes it easier to install and disassemble and more comfortable in use.

In solution II of the present disclosure, a disposable circumcision anastomat is provided, in which it is easier to close and disassemble and more convenient to use an outer ring by improving the structures of binding blocks.

Referring to FIGS. 1-I and 2-I, a disposable circumcision anastomat according to the present disclosure includes an outer ring 1 and an inner ring 2 that match each other. The outer ring 1 is of a structure of which two ends are openable and closed in a snap-fit manner. A first binding block 101 and a second binding block 102 that match each other are provided at the two ends of the outer ring 1 respectively, and at least one of the first binding block 101 and the second binding block 102 is provided with an elastic groove 104.

Specifically, the first binding block 101 and the second binding block 102 are provided one above the other. For example, the first binding block 101 is located on an upper half of the outer ring 1 and the second binding block 102 is located on a lower half of the outer ring 1. The first binding block 101 and the second binding block 102 are provided with matching snap-fit portions 103, for example, in a stepped and serrated form, on their opposite side faces, with serrations having a gentler slope on a side opposite to the opening of the outer ring 1 and a steeper slope on a side facing outward, such that they can slide into each other when the outer ring 1 is closed, can be snap-fitted and fixed by means of engagement of the serrations and grooves, and will not separate after fixation. The elastic groove 104 is located on an inward side of the first binding block 101 or the second binding block 102, and runs through the first binding block 101 or the second binding block 102 lengthwise (in a tangent direction of the outer ring 1). The elastic groove 104 is used to reduce the thickness of the portion(s) of the first binding block 101 and/or the second binding block 102 that is/are connected to the outer ring 1, in order to form a cantilever structure of the elastic connecting plates 105 between the snap-fit portions 103 and the two ends of the outer ring 1, thereby enabling elastic deformation to occur more easily. For example, the elastic connecting plate 105 in the first binding block 101 is located above and the elastic connecting plate 105 in the second binding block 102 is located below, which is conducive to space utilization, and ensures that the elastic connecting plates 105 have a sufficient thickness and the stepped and serrated shape has a sufficiently large undulation to guarantee steady connection, while the height of the outer ring 1 is as small as possible. Preferably, the first binding block 101 and the second binding block 102 are each provided with the elastic groove 104.

Compared with the related art, the elastic connecting plates 105 are relatively thin and thus more elastic, making it easier for the binding blocks to move up and down to a certain extent under the action of an external force, such that the two snap-fit portions 103 slide into each other and are fixed in a snap-fit manner; and after the snap-fit portions 103 are snap-fitted in place, the elastic connecting plates 105 return to their original positions, providing a sufficient force to ensure that the outer ring 1 does not fall apart. The engagement of the stepped and serrated snap-fit portions 103 is a one-way operation, after which the disposable circumcision anastomat cannot be opened again and can only be disassembled destructively after use, thereby ensuring disposability. In order to facilitate disassembling, referring to FIGS. 3-I, the elastic connecting plate(s) 105 of the first binding block 101 and/or the second binding block 102 is/are provided with an unlocking hole 116 running through up and down. During disassembling, an unlocking device such as a thin rod is inserted into the unlocking hole 116 to pry and destroy one of the binding blocks (e.g., to break one elastic connecting plate 105), thereby opening the disposable circumcision anastomosis. Compared with the related art, this solution makes disassembling more labor-saving and convenient, and avoids the difficulty in disassembling.

Further, the outer ring 1 may be of an integral C-shaped structure made of a soft material (such as soft plastic) (as shown in FIG. 3-I), and is opened and closed by virtue of its softness; or the outer ring 1 may be of a movably connected two-segment or multi-segment structure (as shown in FIG. 1-I and FIG. 2-I), with each arc-shaped segment rotatably connected via a pivot structure to achieve opening and closing.

At least one circle of blunt blade 106 for squeezing a prepuce is arranged on the inner side of the outer ring 1. The blunt blade 106 is of a sheet shape, and the blunt blade 106 forms a ring shape after the outer ring 1 is closed. The thickness of the blunt blade 106 gradually decreases from the outside in, thereby forming an edge 109 on the inner side. An upper side face and a lower side face of the blunt blade 106 are preferably frosted faces, in order to prevent adhesion. The blunt blade 106 is provided with an upper half blade extension 113 and a lower half blade extension 114 that match each other at two ends of an opening of the outer ring 1 (as well as at a movably connected portion(s) in the case of a two-segment or multi-segment structure). For example, by halving the thickness at a corresponding position, an upper half of the upper half blade extension 113 is only retained, and a lower half of the lower half blade extension 114 is only retained, such that the two can overlap together during the closing of the outer ring 1. The upper half blade extension 113 and the lower half blade extension 114 have rounded corners at outer ends, such that their inner side edges are smoothly curved during opening and closing, and the prepuce will not be accidentally trapped during closing.

In addition, in order to achieve a better snap-fitting and fixing effect, preferably, the first and second binding blocks and the upper and lower half blade extensions are staggered. That is, as shown in FIG. 1-I, for example, the second binding block 102 is located below, and the upper half blade extension 113 is then adjacent thereto; and at the other end for binding, the first binding block 101 is located above, and the lower half blade extension 114 is located below. In this way, after the outer ring 1 is closed, the upper half blade extension 113 and the lower half blade extension 114 can simultaneously act as a stopper for the first binding block 101 and the second binding block 102, which ensures that the upper and lower half blade extensions fit each other up and down, while the binding blocks are prevented from slippage and separation.

Furthermore, as shown in FIG. 9-I, the disposable circumcision anastomat according to the present disclosure may be circular or oval, with the outer ring 1 and the inner ring 2 matching in shape.

In another aspect, in addition to the integral annular structure shown in FIG. 1-I, the inner ring 2 may adopt a two-segment (or multi-segment) arc-shaped structure as shown in FIG. 8A-I. This avoids the discomfort of a patient caused by the fixed inner diameter of the inner ring 2, and is more convenient for installation and removal.

As a further main improvement of the present disclosure, the edge 109 of the outer ring 1 is provided with a plurality of inwardly protruding protrusions 110 (e.g., ten protrusions are uniformly distributed, which may be specifically adjusted and designed as required), and the inner ring 2 is provided with a plurality of first pits 201 at positions corresponding to the protrusions 110.

Referring to FIGS. 4A-I and 4B-I, the first pits 201 may be blind holes or through holes, and serve to accommodate at least some of the protrusions 110, such that the protrusions 110 can penetrate through inner and outer preputial layers after the outer ring 1 is installed. At a position where there is no protrusion 110 on the blunt blade 106, a certain distance is kept between the edge 109 and the inner ring 2 to form a gap for accommodating the prepuce. In the solutions of the related art, the inner ring has no pits, and the prepuce can only be pushed against a side wall of the inner ring, while the prepuce in the structures of the outer ring and the inner ring is completely closed, which is prone to cause edema. In this solution, the first pits 201 arranged on an outer side of the inner ring 2 and the protrusions 110 on the blunt blade 106 match each other in one-to-one correspondence to penetrate through the inner and outer preputial layers, such that lymph between the inner and outer preputial layers may exude, which is not prone to edema and accelerates healing of a preputial wound.

Further specifically, as shown in FIG. 1-I, for example, the inner ring 2 has for example a ring shape and is provided with an upper flange 204 and a lower flange 206 respectively protruding outwards at two ends, and the prepuce is clamped and fixed between an inner ring sidewall 205 therebetween and the blunt blade 106, which is more stable. In some embodiments, the inner ring 2 further includes a spring gasket 202 (the spring gasket 202 and the inner ring 2 may be of a split structure or an integral structure) arranged on the outer side of the inner ring. The spring gasket is for example made of a rubber material, which has greater friction and better stability. The spring gasket 202 is provided with second pits 203 at positions corresponding to the protrusions 110. The second pits 203 may be blind holes or through holes.

It should be noted that the action principle of the second pits 203 is basically the same as that of the first pits 201, and therefore, in the embodiment having the spring gasket 202, the second pits 203 and the first pits 201 may be regarded as the same structure. For example, in the embodiment shown in FIG. 5A-I, the first pits 201 on the inner ring 2 are the second pits 203 on the spring gasket 202, which is equivalent to setting, based on the embodiment shown in FIG. 4A-I, an outer side face of the inner ring 2 to be elastic, and the spring gasket 202 and the inner ring 2 to be of an integral structure. Alternatively, as shown in FIG. 5B-I, based on the embodiment shown in FIG. 5A-I, the second pits 203 and the first pits 201 are of different structures, and when in use, the spring gasket 202 is pressed, by virtue of its elasticity, into the first pits 201 formed in the body of the inner ring 2. Further, since the spring gasket 202 is relatively thin, it can be envisaged that the second pits 203 are not formed on the spring gasket, and the spring gasket is pressed into the first pits 201 only by virtue of its elasticity, thus omitting the corresponding processing procedure, improving the production efficiency and reducing the cost. Similarly, in the embodiment shown in FIG. 5C-I, the second pits 203 are through holes, but there is no pit on the body of the inner ring 2, and the second pits 203 form the blind-hole type first pits 201. As shown in FIG. 5D-I, based on the embodiment shown in FIG. 5C-I, the body of the inner ring 2 is also separately provided with the first pits 201. For example, the first pits 201 on the body of the inner ring 2 are further in a form of through holes, etc. These conceivable variants are all available.

Further, referring to FIGS. 2-I and 8A-I, the outer ring 1 is provided with protrusion indicators 115 at positions corresponding to the protrusions 110, and/or the inner ring 2 is provided with pit indicators 208 at positions corresponding to the first pits 201 (or second pits 203). Preferably, both the outer ring 1 and the inner ring 2 have corresponding structures thereon. For the case of the outer ring 1, the protrusion indicators 115 may be arranged on an upper face or outer side face of an outer cylinder and/or an upper face of the blunt blade 106, etc. For the case of the inner ring 2, the pit indicators 208 may be arranged on an upper face and/or outer side face of the upper flange 204, etc. The protrusion indicators 115 and the pit indicators 208 may be in any form of marking, such as a relief form of projections and grooves, or simply a form of carving on the face. The protrusion indicators 115 and the pit indicators 208 play a role of facilitating observation during installation, thereby helping a user to align the positions of the protrusions and pits more quickly.

In a further preferred embodiment of the present disclosure, additional bumps are added above and/or below the edge 109. For example, as shown in FIGS. 2-I and 6-I, an upper additional bump 111 and a lower additional bump 112 are arranged above the edge 109 and below the protrusions 110, respectively. The shapes of the first pits 201 and/or second pits 203 correspond to the protrusions 110 and the additional bumps. The additional bumps and the protrusions 110 have such a length in a radial direction that they can be inserted into the second pits 203 of the spring gasket 202, or that they can protrude out of the second pits 203 of the spring gasket 202 from the outside in (with their ends entering the first pits 201 in the body of the inner ring 2). That is, compared with the foregoing embodiments, the inward protruding portion of the edge 109 is changed from a point shape to a vertical line or face shape, which better serves to penetrate through the inner and outer preputial layers to promote lymph exuding, thereby solving the problem of edema occurring during the total confinement operation of circumcision. Preferably, the heights of the additional bumps (the height HA of the upper additional bump and the height HB of the lower additional bump) are greater than the thickness W of the edge of the blunt blade and the heights of the protrusions 110, such that the effects of the additional bumps are more significant. Preferably, the additional bumps have the same sectional shape as the protrusions 110 and are triangular, conical, polygonal, regular quadrilateral (as shown in FIGS. 2-I and 9-I) or U-shaped (as shown in FIG. 3-I). It should be noted that the additional bumps may include the upper additional bump 111 and the lower additional bump 112, or either of the two, and the upper additional bump 111 and/or the additional bump 112 may be staggered instead of being vertically aligned with the protrusions 110 as shown in FIG. 2.

In some further embodiments of the present disclosure, referring to FIG. 7-I, at least two circles of blunt blades (including, for example, an upper blade 107 and a lower blade 108) are arranged on the inner side of the outer ring 1 to form a double-blade (or multi-blade) structure to strengthen the blocking effect on the prepuce. Moreover, a space having a certain volume is naturally formed between the two or more blades, and the space can be used for holding cotton wool, ointment, or medicinal solution, so as to eliminate inflammation and accelerate healing. For the case where both the upper blade 107 and the lower blade 108 (or more blades) are provided with the protrusions 110, the inner ring 2 is correspondingly provided with two (or more) rows of first pits 201 above and below. Further preferably, as shown in FIGS. 8A-I and 8B-I, the inner ring 2 is provided with central projections 207 at positions corresponding to the positions between the two (or more) rows of blades, which form, with the upper flange 204 and the lower flange 206, grooves corresponding to the double blades (or more blades). This further enhances the effect of clamping, fixing and blocking the prepuce to be removed, resulting in necrosis due to poor blood flow.

In summary, the disposable circumcision anastomat according to the present disclosure at least has the following beneficial effects.
1. In the disposable circumcision anastomat according to the present disclosure, the elastic grooves 104 are preferably arranged on the first binding block 101 and the second binding block 102, such that the elastic connecting plates 105 are formed between the snap-fit portions 103 and the two ends of the outer ring 1. Compared with the solutions in the related art, this solution can make it easier, by means of the elastic connecting plates 105, for the snap-fit portions 103 to elastically deform on the premise of ensuring the fixing effect of the outer ring 1, such that it is easier to snap-fit, fix and disassemble the outer ring 1, and it is also easier and more convenient to install and use the outer ring.
2. The disposable circumcision anastomat according to the present disclosure is preferably provided with the protrusions 110 on the outer ring 1 and the pits on the inner ring 2. Therefore, when in use, the disposable circumcision anastomat can penetrate through inner and outer preputial layers, such that lymph between the inner and outer preputial layers may exude, which is not prone to edema and accelerates healing of a preputial wound.
3. In the disposable circumcision anastomat according to the present disclosure, the inner ring 2 is preferably of a two-segment or multi-segment structure, which makes it easier to install and disassemble and more comfortable in use.

In solution III of the present disclosure, a disposal circumcise anastomat is provided, which, by improving the structural design of an edge on an inner side of a blunt blade, achieves the effects of draining lymph and effectively avoiding edema and can accelerate healing of a preputial wound.

Referring to FIGS. 1-II and 2-II, a disposable circumcision anastomat according to an embodiment of the present disclosure includes an outer ring 1 and an inner ring 2 that match each other, and a blunt blade 11 for squeezing a prepuce is arranged on an inner side of the outer ring 1. The blunt blade 11 has a sheet shape, and the blunt blade 11 forms a ring shape after the outer ring 1 is closed. The thickness of the blunt blade 11 gradually decreases from the outside in, thereby forming an edge 12 on an inner side. The edge 12 on the inner side of the blunt blade is provided with a plurality of pits 13 (e.g., ten pits are uniformly distributed, which may be specifically adjusted and designed as required). The pits 13 are preferably either circular point-like pits as shown in FIG. 1-II, or longitudinal strip-shaped grooves as shown in FIG. 4-II, and the pits 13 preferably have a depth of 2 mm. Regardless of their shapes, the pits only occupy a very small portion of the blade, without any effect on the integrity of the blade. In the solutions of the related art, the blunt blade 11 has no pits 13, and the prepuce in the structures of the outer ring 1 and the inner ring 2 is completely closed, and thus is prone to edema. The pits 13 arranged according to the solutions of the present disclosure have the effect of draining the lymph to effectively prevent edema.

For example, the inner ring 2 has for example a ring shape and is provided with an upper flange 24 and a lower flange 26 respectively protruding outwards at two ends, and the prepuce is clamped and fixed between an inner ring sidewall 25 therebetween and the blunt blade 11, which is more stable. Preferably, a plurality of protrusions 21 are arranged on an outer side of the inner ring 2, and the protrusions 21 and the pits 13 are in one-to-one correspondence. In this way, when in use, the protrusions 21 enter the pits 13, and the inner and outer preputial layers are then penetrated through to allow for exuding of lymph between the inner and outer preputial layers, which further prevents the lymph from accumulation. At a position where there is no protrusion 13 on the blunt blade 11, a certain distance is kept between the edge 12 and the inner ring 2 to form a gap for accommodating the prepuce.

Referring to FIG. 3-II, in some embodiments, the inner ring 2 further includes a spring gasket 22 (the spring gasket 22 and the inner ring 2 may be of a split structure or an integral structure) arranged on the outer side of the inner ring. The spring gasket is for example made of a rubber material, which has greater friction and better stability. The spring gasket 22 is provided with gasket pits 23 at positions corresponding to the pits 13, and the gasket pits 23 are preferably through holes, which are communicated to the pits 13.

Further preferably, the outer ring and/or the inner ring is/are provided with indicators at positions corresponding to the pits. As shown in FIG. 1-II, the outer ring 1 is provided with outer ring indicators 14 at positions corresponding to the pits 13; and as shown in FIG. 5-II, the inner ring 2 is provided with inner ring indicators 28 at positions corresponding to the pits 13 (or protrusions 21). Preferably, both the outer ring 1 and the inner ring 2 have indicator structures thereon. For the case of the outer ring 1, the outer ring indicators 14 may be arranged on an upper face or outer side face of an outer cylinder and/or an upper face of the blunt blade, etc. For the case of the inner ring 2, the pit indicators 28 may be arranged on an upper face and/or outer side face of the upper flange 24, etc. The outer ring indicators 14 and the inner ring indicators 28 may be in any form of marking, such as a relief form of projections and grooves, or simply a form of carving on the face. The outer ring indicators 14 and the inner ring indicators 28 serve to facilitate observation and alignment during installation.

Referring to FIG. 2-II, at least one circle of blunt blade 11 is arranged on the inner side of the outer ring 1, and an upper side face and a lower side face of the blunt blade 11 are preferably frosted faces, in order to prevent adhesion. An upper half blade extension 15A and a lower half blade extension 15B that match each other are arranged at two ends of an opening of the outer ring 11 (as well as at a movably connected portion(s) in the case of a two-segment or multi-segment structure). For example, by halving the thickness at a corresponding position, an upper half of the upper half blade extension 15A is only retained, and a lower half of the lower half blade extension 15B is only retained, such that the two can overlap together during the closing of the outer ring 1. The upper half blade extension 15A and the lower half blade extension 15B have rounded corners 16 at outer ends, such that their inner side edges are smoothly curved during opening and closing, and the prepuce will not be accidentally trapped during closing.

A first binding block 17A and a second binding block 17B are arranged at two ends of the outer ring 1, respectively. The first and second binding blocks are arranged with a height difference and provided with serrations on their opposing faces. The serrations have a gentler slope on a side opposite to the opening of the outer ring 1 and a steeper slope on a side facing outward, such that they can be snap-fitted and fastened when the opening is closed and will not separate easily (the first binding block 17A and the second binding block 17B can be pried apart by a hard rod-type tool after the use of the disposable circumcision anastomat). Preferably, at least one of the first binding block 17A and the second binding block 17B is provided with an elastic groove 18, which is used to reduce the thickness of the portion(s) of the first binding block 17A and/or second binding block 17B that is/are connected to the outer ring 1, such that the portion(s) is/are more susceptible to elastic deformation, allowing for easier snap-fit when the outer ring 1 is installed and closed. In addition, in order to achieve a better snap-fitting and fixing effect, preferably, the first and second binding blocks and the upper and lower half blade extensions are staggered. That is, as shown in FIG. 1, for example, the first binding block 17A is located below, and the upper half blade extension 15A is then adjacent thereto; and at the other end for binding, the second binding block 17B is located above, and the lower half blade extension 15B is located below. In this way, after the outer ring 1 is closed, the upper half blade extension 15A and the lower half blade extension 15B can simultaneously act as a stopper for the first binding block 17A and the second binding block 17B, thereby preventing slippage and separation. It should be noted that snap-fit structures, bolt-screw hole structures or other structures having similar fixing effect are conceivable, which is not limited in the present disclosure.

In some further embodiments of the present disclosure, referring to FIGS. 5-II and 6-II, at least two circles of blunt blades (including, for example, an upper blade 11A and a lower blade 11B) are arranged on the inner side of the outer ring 1 to form a double-blade (or multi-blade) structure to strengthen the blocking effect on the prepuce. Moreover, a space having a certain volume is naturally formed between the two or more blades, and the space can be used for holding cotton wool, ointment, or medicinal solution, so as to eliminate inflammation and accelerate healing. Optionally, either of or both the upper blade 11A and the lower blade 11B (or more blades) is/are provided with pits 13, and the pits 13 on the upper blade 11A and the lower blade 11B (or more blades) may be aligned or staggered vertically. Also, the inner ring 2 is optionally provided with two (or more) rows of protrusions 21 above and below correspondingly. The inner ring 2 is provided with central projections 27 at positions corresponding to the positions between the two (or more) rows of blades, which form, with the upper flange 24 and the lower flange 26, grooves corresponding to the double blades (or more blades). This further enhances the effect of clamping, fixing and blocking the prepuce to be removed, resulting in necrosis due to poor blood flow.

As shown in FIG. 7-II, the outer ring 1 may be of an integral C-shaped structure made of a soft material (such as soft plastic), and is opened and closed by virtue of its softness; or as shown in FIGS. 2-II and 1-II, the outer ring 1 may be of a movably connected two-segment, three-segment or multi-segment structure, with each arc-shaped segment rotatably connected via a pivot structure to achieve opening and closing.

In another aspect, in addition to the integral annular structure shown in FIG. 1, the inner ring 2 may adopt a two-segment (or multi-segment) arc-shaped structure as shown in FIG. 6-II. This avoids the discomfort of a patient caused by the fixed inner diameter of the inner ring 2, and is more convenient for installation and removal.

Furthermore, as shown in FIG. 9-II, the disposable circumcision anastomat according to the present disclosure may be circular or oval, with the outer ring 1 and the inner ring 2 matching in shape.

In summary, the disposable circumcision anastomat of the present disclosure at least has the following beneficial effects.

In the disposable circumcision anastomat according to the present disclosure, the pits 13 are formed on the inner side of the blunt blade 11 of the outer ring 1 to play a role of draining lymph, such that edema can be effectively prevented and healing of a preputial wound can be accelerated. Preferably, the inner ring 2 is further provided with protrusions 21. Therefore, when in use, the disposable circumcision anastomat can penetrate through inner and outer preputial layers, such that lymph between the inner and outer preputial layers may exude, which further prevents lymph from accumulation. Moreover, the outer ring 1 and the inner ring 2 have indicators corresponding to the pits 13 to facilitate observation and alignment during installation. In addition, the inner ring 21 is preferably of a two-segment or multi-segment structure, which makes it easier to install and disassemble and more comfortable in use.

## Claims

1. A disposable circumcision anastomat, comprising an outer ring and an inner ring that match each other, the outer ring being provided with a blunt blade for squeezing a prepuce, and an edge of the blunt blade being provided with a plurality of protrusions, wherein the inner ring is provided with first pits at positions corresponding to the protrusions.

2. The disposable circumcision anastomat according to claim 1, wherein the first pits are through holes.

3. The disposable circumcision anastomat according to claim 1 or 2, wherein a spring gasket is arranged on an outer side of the inner ring, and the spring gasket is provided with second pits at positions corresponding to the protrusions.

4. The disposable circumcision anastomat according to claim 3, wherein the second pits are through holes.

5. The disposable circumcision anastomat according to claim 1, wherein additional bumps are added above and/or below the edge of the blunt blade.

6. The disposable circumcision anastomat according to claim 5, wherein heights of the additional bumps are greater than a thickness of the edge of the blunt blade.

7. The disposable circumcision anastomat according to claim 6, wherein the additional bumps have a same shape as the protrusions and are triangular, conical, polygonal, regular quadrilateral or U-shaped.

8. The disposable circumcision anastomat according to claim 6, wherein a spring gasket is arranged on the outer side of the inner ring, the spring gasket is provided with second pits at positions corresponding to the protrusions, and the additional bumps and the protrusions are inserted into the second pits of the spring gasket or protrude out of the second pits of the spring gasket.

9. The disposable circumcision anastomat according to any one of claims 1-2 and 5-8, wherein the outer ring is of an integral C-shaped structure made of a soft material, or is of a movably connected two-segment or multi-segment structure.

10. The disposable circumcision anastomat according to any one of claims 1-2 and 5-8, wherein at least one circle of annular blunt blade is arranged on an inner side of the outer ring, an upper half blade extension and a lower half blade extension that match each other are arranged at two ends of an opening of the outer ring, and the upper half blade extension and the lower half blade extension have rounded corners at outward end portions.

11. The disposable circumcision anastomat according to any one of claims 1-2 and 5-8, wherein a first binding block and a second binding block that match each other are provided at two ends of the outer ring respectively, and at least one of the first binding block and the second binding block is provided with an elastic groove.

12. The disposable circumcision anastomat according to any one of claims 1-2 and 5-8, wherein the inner ring is of an integral annular structure, or is of a two-segment or multi-segment arc-shaped structure.

13. The disposable circumcision anastomat according to any one of claims 1-2 and 5-8, wherein at least two layers of annular blunt blades are arranged on the inner side of the outer ring.

14. The disposable circumcision anastomat according to any one of claims 1-2 and 5-8, wherein the disposable circumcision anastomat is circular or oval.

15. The disposable circumcision anastomat according to any one of claims 1-2 and 5-8, wherein an upper side face and a lower side face of the blunt blade of the outer ring are frosted faces.

16. The disposable circumcision anastomat according to any one of claims 1-2 and 5-8, wherein the outer ring is provided with protrusion indicators at positions corresponding to the protrusions, and/or the inner ring is provided with pit indicators at positions corresponding to the first pits.

17. A disposable circumcision anastomat, comprising an outer ring and an inner ring that match each other, the outer ring being of a structure of which two ends are openable and closed in a snap-fit manner, wherein a first binding block and a second binding block that match each other are provided at the two ends of the outer ring respectively, and at least one of the first binding block and the second binding block is provided with an elastic groove.

18. The disposable circumcision anastomat according to claim 17, wherein the first binding block is located at an upper portion of the outer ring and the second binding block is located at a lower portion of the outer ring; snap-fit portions that match each other are arranged on opposite side faces of the first binding block and the second binding block; and the elastic groove is located on an inward side of the first binding block or the second binding block and runs through the first binding block or the second binding block lengthwise, and an elastic connecting plate is formed between the snap-fit portions and the two ends of the outer ring.

19. The disposable circumcision anastomat according to claim 18, wherein the elastic connecting plate in the first binding block is located above, and the elastic connecting plate in the second binding block is located below.

20. The disposable circumcision anastomat according to claim 18, wherein the snap-fit portion is stepped and serrated.

21. The disposable circumcision anastomat according to claim 17, wherein the outer ring is of an integral C-shaped structure made of a soft material, or is of a movably connected two-segment or multi-segment structure.

22. The disposable circumcision anastomat according to claim 17, wherein at least one circle of annular blunt blade is arranged on an inner side of the outer ring, an upper half blade extension and a lower half blade extension that match each other are arranged at the two ends of the outer ring, and the upper half blade extension and the lower half blade extension have rounded corners at outward end portions.

23. The disposable circumcision anastomat according to any one of claims 17-22, wherein an inner side of the blunt blade of the outer ring is an edge, the edge is provided with a plurality of protrusions, and the inner ring is provided with first pits at positions corresponding to the protrusions.

24. The disposable circumcision anastomat according to claim 23, wherein a spring gasket is arranged on an outer side of the inner ring, and the spring gasket is provided with second pits at positions corresponding to the protrusions.

25. The disposable circumcision anastomat according to claim 24, wherein the first pits and/or the second pits are through holes.

26. The disposable circumcision anastomat according to claim 23, wherein additional bumps are added above and/or below the edge.

27. The disposable circumcision anastomat according to claim 23, wherein the outer ring is provided with protrusion indicators at positions corresponding to the protrusions, and/or the inner ring is provided with pit indicators at positions corresponding to the pits.

28. The disposable circumcision anastomat according to any one of claims 17-22, wherein the inner ring is of an integral annular structure, or is of a two-segment or multi-segment arc-shaped structure.

29. The disposable circumcision anastomat according to any one of claims 17-22, wherein at least two layers of annular blunt blades are arranged on the inner side of the outer ring.

30. The disposable circumcision anastomat according to any one of claims 17-22, wherein the disposable circumcision anastomat is circular or oval.

31. The disposable circumcision anastomat according to any one of claims 17-22, wherein an upper side face and a lower side face of the blunt blade of the outer ring are frosted faces.

32. A disposable circumcision anastomat, comprising an outer ring and an inner ring that match each other, the outer ring being provided with a blunt blade for squeezing a prepuce, wherein a plurality of pits are circumferentially arranged on an inner side of the blunt blade.

33. The disposable circumcision anastomat according to claim 32, wherein the pits are either circular point-like pits or longitudinal strip-shaped grooves.

34. The disposable circumcision anastomat according to claim 32, wherein the pits have a depth of 2 mm.

35. The disposable circumcision anastomat according to claim 32, wherein a plurality of protrusions are arranged on an outer side of the inner ring, and the protrusions and the pits are in one-to-one correspondence.

36. The disposable circumcision anastomat according to claim 32, wherein a spring gasket is arranged on the outer side of the inner ring.

37. The disposable circumcision anastomat according to claim 32, wherein the spring gasket is provided with gasket pits at positions corresponding to the pits.

38. The disposable circumcision anastomat according to claim 37, wherein the gasket pits are through holes.

39. The disposable circumcision anastomat according to any one of claims 32-38, wherein the outer ring is of an integral C-shaped structure made of a soft material, or is of a movably connected two-segment or multi-segment structure.

40. The disposable circumcision anastomat according to any one of claims 32-38, wherein at least one circle of annular blunt blade is arranged on an inner side of the outer ring, an upper half blade extension and a lower half blade extension that match each other are arranged at two ends of an opening of the outer ring, and the upper half blade extension and the lower half blade extension have rounded corners at outward end portions.

41. The disposable circumcision anastomat according to any one of claims 32-38, wherein a first binding block and a second binding block that match each other are provided at the two ends of the outer ring respectively, and at least one of the first binding block and the second binding block is provided with an elastic groove.

42. The disposable circumcision anastomat according to any one of claims 32-38, wherein the inner ring is of an integral annular structure, or is of a two-segment or multi-segment arc-shaped structure.

43. The disposable circumcision anastomat according to any one of claims 32-38, wherein at least two layers of annular blunt blades are arranged on the inner side of the outer ring.

44. The disposable circumcision anastomat according to any one of claims 32-38, wherein the disposable circumcision anastomat is circular or oval.

45. The disposable circumcision anastomat according to any one of claims 32-38, wherein an upper side face and a lower side face of the blunt blade of the outer ring are frosted faces.

46. The disposable circumcision anastomat according to any one of claims 32-38, wherein the outer ring and/or the inner ring is/are provided with indicators at positions corresponding to the pits.
